# EUROPEAN PATENT SPECIFICATION

(11) **EP 1 402 894 B1**
(45) Date of publication and mention of the grant of the patent: **26.12.2007**
(21) Application number: 02771731.3
(22) Date of filing: 20.05.2002
(51) Int. Cl.: A61K 31/662, A61P 35/04, C07F 9/6574

(54) **CANCEROUS METASTASIS INHIBITORS CONTAINING CARBACYCLIC PHOSPHATIDIC ACID DERIVATIVES**
KREBSMETASTASEN-HEMMER MIT CARBACYCLISCHEN PHOSPHATIDINSÄUREDERIVATEN
INHIBITEURS DE METASTASES CANCEREUSES CONTENANT DES DERIVES D'ACIDE PHOSPHATIDIQUE CARBACYCLIQUES

(30) Priority: 21.05.2001 JP 2001150685
(43) Date of publication of application: 31.03.2004
(73) Proprietor: Murofushi, Kimiko, Saitama-shi, Saitama 336-0026 (JP)
(72) Inventor: MUKAI, Mutsuko, Koube-shi, Hyogo 657-0024 (JP); KOBAYASHI, Susumu, Meguro-ku, Tokyo 152-0032 (JP); MUROFUSHI, Hiromu, Saitama-shi, Saitama 336-0026 (JP); MUROFUSHI, Kimiko, Saitama-shi, Saitama 336-0026 (JP)
(74) Representative: Polypatent
(86) International application number: PCT/JP2002/004839
(87) International publication number: WO 2002/094286

(56) References cited:
- WO-A2-00/57864
- JP-A- 6 228 169
- JP-A- 9 025 235
- PATENT ABSTRACTS OF JAPAN vol. 1996, no. 02, 29 February 1996 (1996-02-29) & JP 07 258278 A (SAGAMI CHEM RES CENTER), 9 October 1995 (1995-10-09)
- YOKOMATSU,T. ET AL.: 'Electrophilic heteroatom cyclization of omega-alkenylphosphonic acid half esters giving cyclic phosphonates (phostones)' HETEROCYCLES vol. 46, 1997, pages 463 - 472, XP002955808
- BESTMANN, H.J. ET AL.: 'Phosphine alkylenes. 50. On the structure of 2,2,2,-triphenyl-1,2 lambda 5-oxaphospholanes' CHEMICAL BER. vol. 125, no. 1, 1992, pages 225 - 229, XP002955809
- DATABASE CAPLUS [Online] 1986 WROBLEWSKI A.E., XP002957312 Database accession no. 1986:591524 & WROBLEWSKI, A.E. Z. NATURFORSCH. vol. 41B, no. 6, 1986, pages 791 - 792
- MUKAI ET AL: "Inhibition of tumor invasion and metastasis by a novel lysophosphatidic acid (cyclic LPA)" INT. J. CANCER, vol. 81, no. 6, 1999, pages 918-922, XP000949280

## Description

### TECHNICAL FIELD

The present invention relates to an agent for suppression of cancer metastasis which contains a carbacyclic phosphatidic acid derivative as an active ingredient.

### BACKGROUND OF THE INVENTION

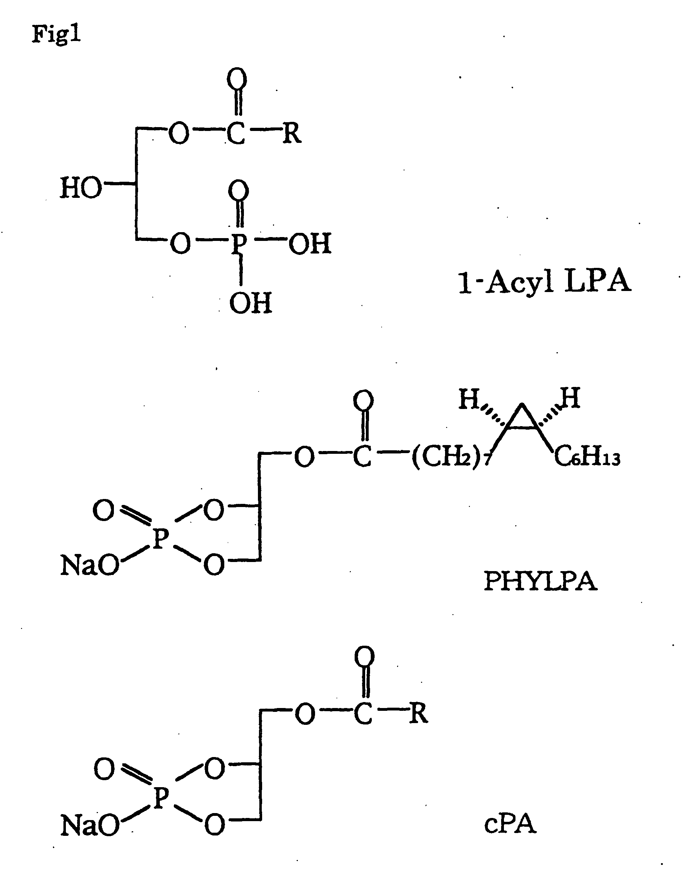

Lysophosphatidic acid (LPA) has the simplest structure among phospholipids which compose the biological membrane, wherein either fatty acid at the sn-1 or -2 position of glycerol in phosphatidic acid (PA) is deacylated (Fig.1). In a normal cell, the LPA ratio of all phospholipids is as extremely low as 0.5% or less. By the 1980s, LPA was believed to be merely an intermediate or a degradation product of phospholipid biosynthesis. However, recently, various physiological activities of LPA have been shown (Moolenaar, W. H.: Exp. Cell Res. 253, 230-238 (1999)). Furthermore, the presence of several receptors on the cell membrane has also been revealed (Guo, Z., et al., Proc. Natl. Acad. Sci. USA 93, 14367-14372 (1996); Hecht, J. H., et al., J. Cell Biol. 135, 1071-1083 (1996); An, S., et al., J. Biol. Chem. 273, 7906-7910 (1998); Bandoh, K., et al., J. Biol. Chem. 274, 27776-27785 (1999); and Im, D-S., et al., Mol. Pharmacol. 57, 753-759 (2000)). LPS is now attracting attention as a functional phospholipid.

LPA is known to induce various actions depending on cell type. In addition to the promotion of cell proliferation (van Corven, E. J., et al, Cell 59, 45-54 (1989)) and the suppression of cell death (Umansky, S.R, et al, Cell Death Diff. 4, 608-616 (1997)), LPA guides changes in the cytoskeleton by activating the signal transduction system via Rho which is a low-molecular-weight G protein, and induces the stress fiber formation in fibroblasts (Gohla, A., et al., J. Biol. Chem. 273, 4653-4659 (1998)), the degeneration of neurite in nerve cells (Tigyi, G, et al., J. Biol. Chem. 267, 21360-21367 (1992) ; Jalink, K., et al., Cell Growth & Differ. 4, 247-255 (1993); Jalink, K., et al., J. Cell Biol. 126, 801-810 (1994); and Tigyi, G, et al., J. Neurochem. 66, 537-548 (1996)), the invasion of cancer cell (Imamura, F., et al., Biochem. Biophym. Res. Commun. 193, 497-503 (1993); O'Connor, K. L., et al., J. Cell Biol. 143, 1749-1760 (1998); and Stam, J.C., et al., EMBO J. 17, 4066-4074 (1998)) and the like.

In 1992, a fat-soluble substance that suppresses the activity of DNA polymerase α, a DNA replication enzyme of eukaryotic cells, and suppresses the proliferation of animal culture cells was discovered, isolated and purified from mixoamoeba, the haplont of Physarum polycephalum slime molds (Murakami-Murofushi, K., et al., J. Biol. Chem. 267, 21512-21517 (1992)). It has been shown that, in this substance, hexadecanoic acid containing cyclopropane is bound at the sn-1 position of the glycerol backbone, and phosphoric acid is bound via cyclic ester bond at the sn-2 and -3 positions of the glycerol backbone (Fig.1). This substance is named PHYLPA since it is a Physarum-derived LPA-like substance.

Since PHYLA has a characteristic fatty acid at the sn-1 position, a derivative was chemically synthesized by substituting the fatty acid with a general fatty acid, and its activity was studied. As a result, it was shown that all substances suppress cell proliferation while they differ in their suppression degrees. Thus it was revealed that the anti-proliferative action of PHYLPA results from the cyclic phosphate group at the sn-2 and -3 positions. At present, these LPA analogs having such cyclic phosphate group are generically called cPA, cyclic phosphatidic acid (Fig. 1). cPA has been recently detected in the form of being bound to albumin in a human serum (Kobayashi, T., et al., Life Sciences 65, 2185-2191 (1999)), revealing that cPA is broadly present in the living world. Moreover, the cPA in a lipid fraction separated at this time mainly consists of Pal-cPA having palmitic acid as fatty acid. The presence of cPA has also been confirmed in the rat and pig brains in addition to being present in sera. Tigyi et al has also detected a group of LPA analogues containing cPA in the aqueous humour or lacrimal gland fluid of a rabbit model of corneal damage (Liliom, K., et al, Am. J. Physiol. 274, C1065-C1074 (1998)). cPA has been revealed to exhibit various physiological activities similar to or contrary to those of LPA. While LPA promotes the proliferation of culture cells, cPA exhibits suppression action (Murakami-Murofushi, K., et al, Cell Struct. Funct. 18, 363-370 (1993)). This action is reversible. When cPA is removed from a medium, cells begin to proliferate again. Two possibilities have been suggested as the mechanism for anti-proliferative action.

In mouse fibroblasts (NIH3T3), it has been found that cPA causes a rise in intracellular cAMP concentration within several minutes. This phenomenon disappears by blocking the rise in intracellular Ca²⁺ concentration (Murakami-Murofushi, K., et al, Cell Struct. Funct. 18, 363-370 (1993)), suggesting a possibility that cPA may activate Ca²⁺ -dependent adenylate cyclase via receptors on the cell membrane. It has been shown that a rise in intracellular cAMP concentration suppresses the activation of MAP kinase (Hordijk, P. L., et al, J. Biol. Chem. 269, 3534-3538 (1994); and Howe, L.R, et al, J. Biol. Chem. 268, 20717-20720 (1993)), suggesting that this may lead to the inhibition of proliferation. On the other hand, it is considered that cPA is easily incorporated within cells because of its structure. Regarding this point, cPA having the sn-1 position labeled with fluoresence was synthesized and added to cells, and then its behavior was observed. As a result, it has been revealed that cPA rapidly shifts into the cells, and is localized in the peripheral portion of the nucleus in the cytoplasm. Furthermore, it has also been found that cPA inhibits, in vitro, cdc25 phosphatase activity which controls the cell cycle (Tetsuyuki KOBAYASHI and Kimiko MUROFUSHI: Protein, Nucleic Acid and Enzyme, 44, 1118-1125 (1999)). Accordingly, it is also possible that cPA inhibits the cell proliferation by directly suppressing the activation of cdk2 kinase complex in the cytoplasm without involving receptors on the cell membrane.

Moreover, in fibroblasts cultured in a medium with a limited serum level, both LPA and cPA induce stress fiber formation by actin molecules within the cells (Tetsuyuki KOBAYASHI and Kimiko MUROFUSHI: Protein, Nucleic Acid and Enzyme, 44, 1118-1125 (1999)). In this case, similar to LPA, cPA is considered to initiate its action by activating Rho via the binding to a cell membrane receptor.

Furthermore, regarding the invasion of cancer cells, cPA exhibits strong suppressing activity in contrast to the promotion action of LPA (Mukai, M., et al, Int. J. Cancer 81, 918-922 (1999)).

Cancer metastasis is the most significant phenomenon showing malignancy of tumor, and is established through complex steps. In particular, the invasion is a characteristic step. Cancer cells which were released from the primary lesion in vivo invade stroma and blood vessels. The cells are then transported via the blood stream, invade outside the blood vessels and further invade remote organs, in which the cells then start proliferation to form a metastatic lesion. To analyze in vitro this phenomenon, AKEDO et al have modeled peritoneal invasion that was developed when rat ascites hepatoma cells (AH-130) were implanted in the rat abdominal cavity, and thus have developed an experimental system with which the migration of cancer cells across the normal host cell layer can be quantitatively evaluated (Akedo, H., et al, Cancer Res. 46, 2416-2422 (1986)). Normally, an experimental system for observing cancer cells that pass through the membrane coated with extracellular substrates is used to study invasion. In contrast to such method, this system is thought to well reflect an in vivo state. Some cancer cells to be used for this experimental system need serum but some of them do not need any serum for invasion. A highly invasive clone (MM1) of AH-130 cells requires serum for its invasion. As a result of search for an invasion-promoting substance in serum, it was revealed that LPA is at least one of such invasion-promoting substances (Imamura, F., et al, Biochem. Biophym. Res. Commun. 193, 497-503 (1993)). Accordingly, the effect of cPA, a structural analog of LPA, on the invasion was examined. As a result, it was found that cPA suppresses invasion, completely contrary to the case of LPA. Some derivatives having different fatty acids bound at sn-1 position were synthesized, and their invasion inhibitory activities were examined. As a result, Pal-cPA exhibited the strongest invasion suppressing activity. Furthermore, in the same assay system, Pal-cPA significantly suppressed the invasion of human fibrosarcoma cells (HT-1080) which require neither serum nor LPA for their invasion (Mukai, M., et al, Int. J. Cancer 81, 918-922 (1999)).

Also in MM1 cells, intracellular cAMP concentration was elevated within several minutes by the addition of Pal-cPA, and this effect was not lost even during co-existence with LPA (Mukai, M., et al, Int. J. Cancer 81, 918-922 (1999)). When a reagent which can increase the intracellular cAMP concentration was added, LPA-induced invasion was suppressed. Moreover, it was shown that LPA-mediated Rho activation is inhibited by the addition of these reagents or Pal-cPA (Mukai, M., et al, FEBS Letters 484, 69-73 (2000)). These results suggest that also in this cancer cell (MM1), cPA may inhibit Rho activity via activation of cAMP-dependent Protein Kinase A (A kinase) by increasing an intracellular cAMP concentration, so as to suppress invasion. However, it has not been reported so far whether a carbacyclic phosphatidic acid derivative has an action of suppressing the invasion of cancer cell.

### DISCLOSURE OF THE INVENTION

An object to be achieved by the present invention is to provide a novel agent for suppression of cancer metastasis by studying the suppressing action of cancer cell invasion by carbacyclic phosphatidic acid derivatives.

The present inventors have chemically synthesized 11 types of cPA derivatives according to the method established by KOBAYASHI et al (Kobayashi, S., et al., Tetrahedron Letters 34, 4047-4050 (1993)), and then studied the effect of these derivatives on the serum/LPA-dependent invasion in an in vitro invasion assay system (Example 1 below). As a result, cPA derivatives which exhibit an invasion suppressing activity which is 10 to 100 times greater than that of Pal-cPA were identified, and then the effects thereof on LPA-independent invasion were examined (Example 2 below). Finally, the action and mechanism of the invasion suppressing activity of the cPA derivative (Example 3 below). As a result, it was demonstrated that the carbacyclic phosphatidic acid derivative has an activity of suppressing cancer cell invasion, and thus the present invention has been completed.

Thus, the present invention provides an agent for suppression of cancer metastasis which contains a compound represented by the following formula (I) as an active ingredient: 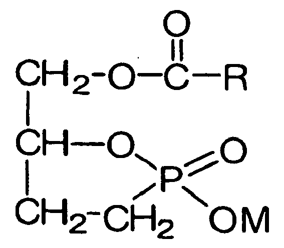 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

According to a particularly preferred embodiment of the present invention, in the formula (I), R represents -C₁₅H₃₁, -(CH₂)₇CH=CHC₆H₁₃ or -(CH₂)₇CH=CHC₈H₁₇.

A particularly preferred embodiment of the present invention provides an agent for suppression of cancer metastasis, which suppresses cancer metastasis by suppressing cancer cell invasion.

According to further another aspect of the present invention, there is provided a method for suppressing cancer metastasis, which comprises administering a therapeutically effective dose of the compound represented by the above formula (I) to a mammal, including a human.

According to further another aspect of the present invention, there is provided a use of the compound represented by the above formula (I) for the production of an agent for suppression of cancer metastasis.

### BRIEF DESCRIPTION OF THE DRAWINGS

FIG. 1 shows the structures of LPA, PHYLPA and cPA.

FIG. 2 shows the structure of a chemically synthesized cPA derivative.

FIG. 3 shows an in vitro invasion assay system.
(a) Method of in vitro invasion assay: Cancer cells were multi-layer-cultured on a normal cell monolayer that has been cultured to reach a confluent state. The cells were fixed after a certain time period, and then observed under a phase contrast microscope.
(b) Images of invading cells, which were observed under the phase contrast microscope. Invading cancer cells (MM1) are indicated by arrows.

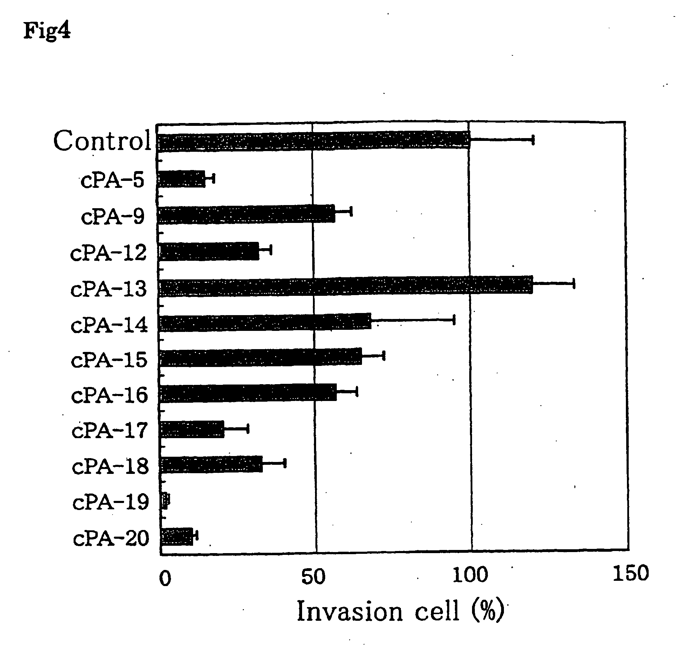

FIG 4 shows the effect of cPA derivative on FBS-induced invasion. 25 µM cPA derivative was added to 1×10⁵ cells/ml MM1 cells in the presence of 10% FBS, and then in vitro invasion assay was performed. The invasion suppressing action by cPA is shown with the ratio (%) to that of a control.

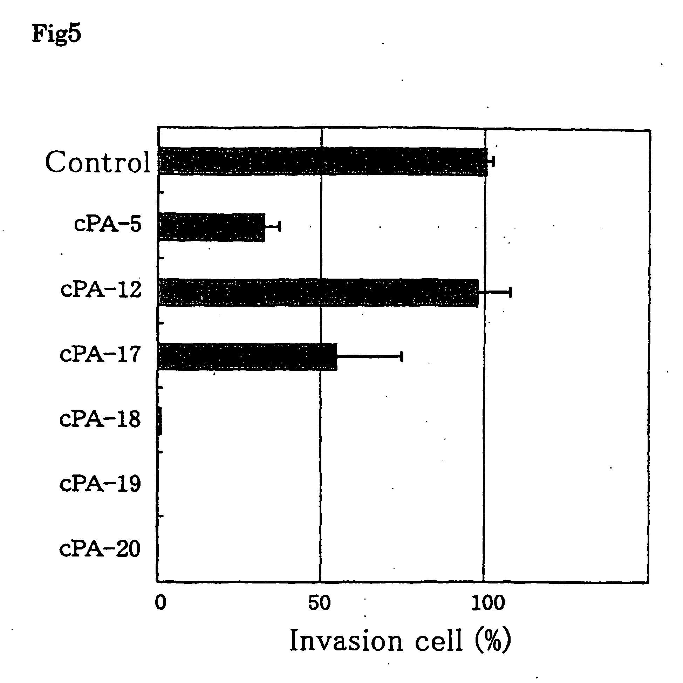

FIG. 5 shows the effect of cPA derivative on LPA-induced invasion. 25 µM cPA derivative was added to 1.5×10⁵ cells/ml MM1 cells in the presence of 25 µM LPA, and then in vitro invasion assay was performed. The invasion suppressing action by cPA is shown with the ratio (%) to that of a control.

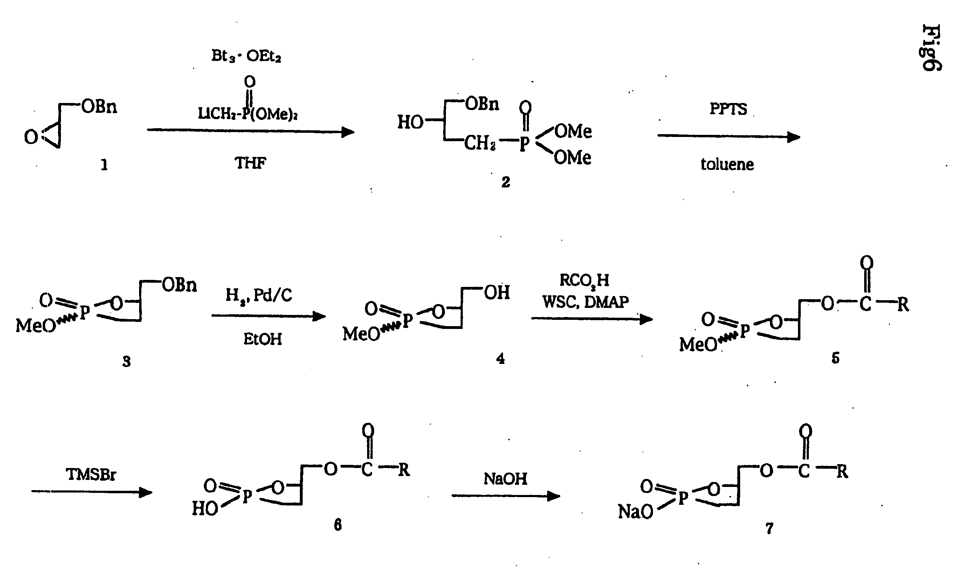

FIG. 6 shows the chemical synthesis pathway of Carba-cPA. Numbers in this figure correspond to the numbers in the explanation for the synthesis given in Example 1 (1-2).

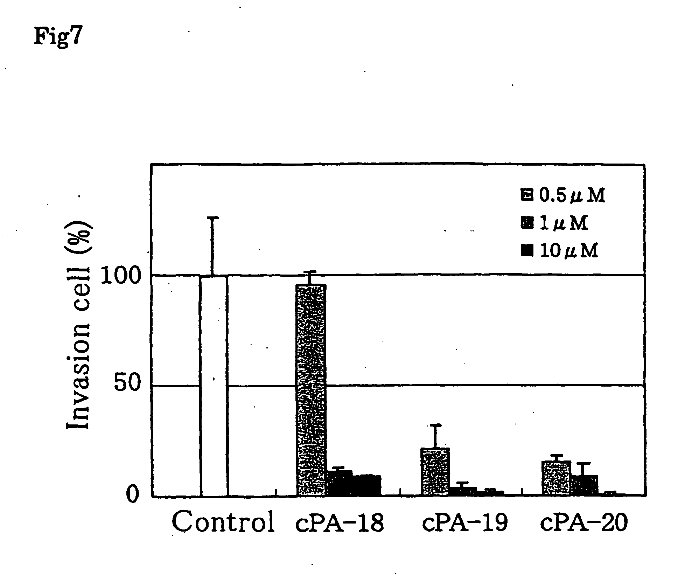

FIG. 7 shows the effect of carba-cPA on LPA-induced invasion.

0.5 µM, 1 µM or 10 µM carba-cPA was added to 1.5×10⁵ cells/ml MM1 cells in the presence of 25 µM LPA, and then in vitro invasion assay was performed. The invasion suppressing action by carba-cPA is shown with the ratio (%) to that of a control.

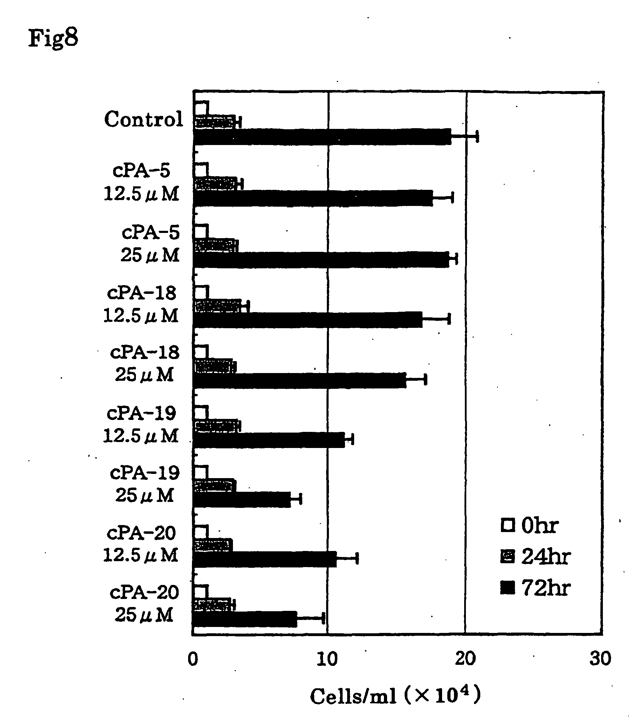

FIG. 8 shows the effect of cPA on the proliferation of MM1 cells.

12.5 µM or 25 µM cPA was added to 1×10⁴ cells/ml MM1 cells in the presence of 10% FBS, and then the cells were cultured. The number of the cells was counted at 24 hours and 72 hours after culture.

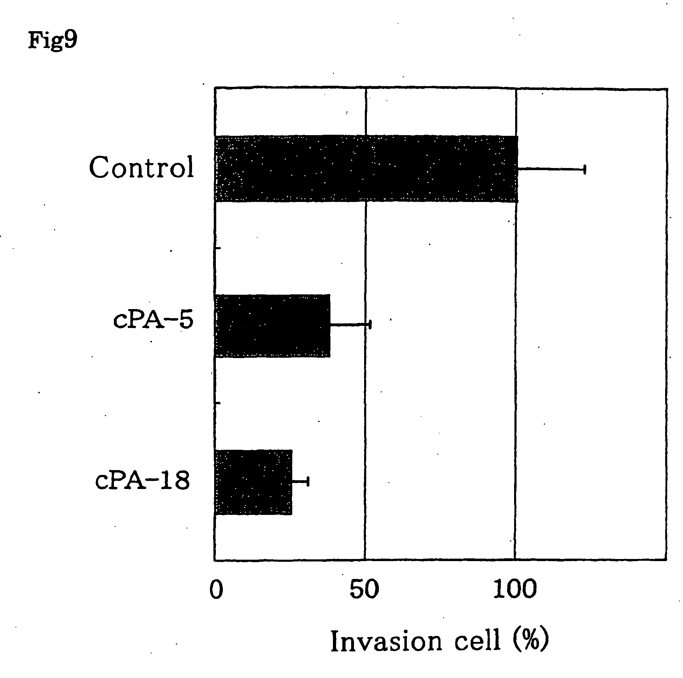

FIG. 9 shows the effect of cPA on the invasion of HT-1080 cells.

10 µM cPA was added to 2×10⁵ cells/ml HT-1080 cells in the absence of serum/LPA, and then in vitro invasion assay was performed. The invasion suppressing action by cPA is shown with the ratio (%) to that of a control.

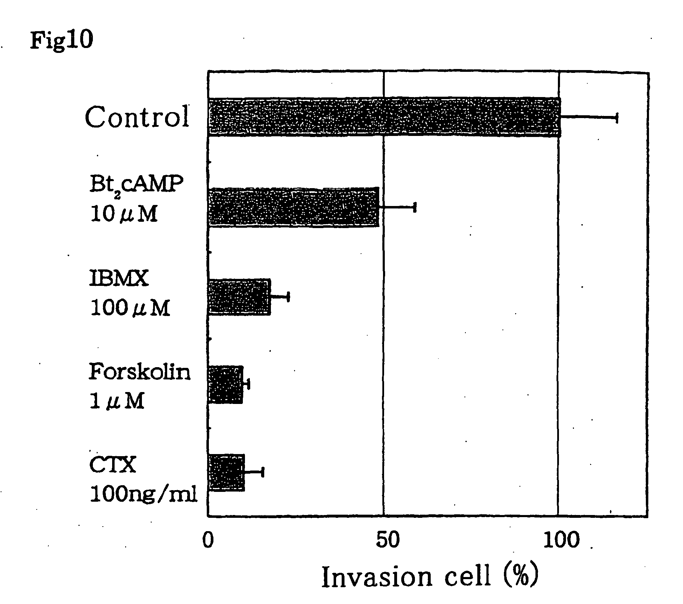

FIG. 10 shows the effect of a reagent for increasing cAMP on the LPA-induced invasion.

The reagent for increasing intracellular cAMP concentration was added at concentrations as shown in the figure to 1.5×10⁵ cells/ml MM1 cells in the presence of 25 µM LPA, and then in vitro invasion assay was performed. The invasion suppressing actions by them are shown with the ratio (%) to that of control.

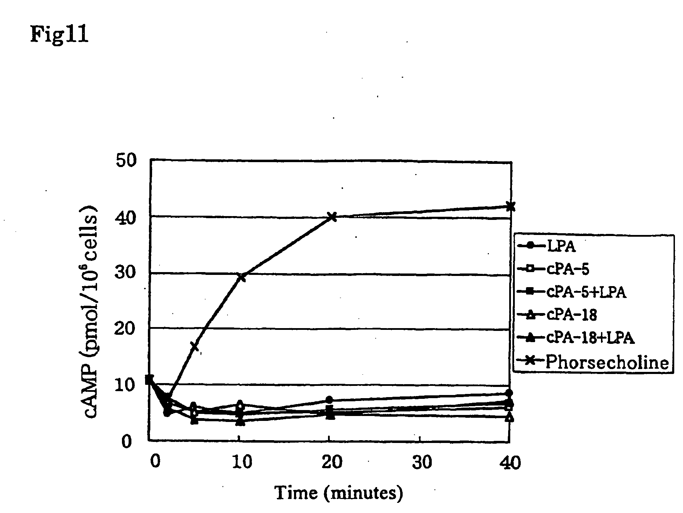

FIG. 11 shows the effect of cPA on intracellular cyclic AMP.

25µM cPA was added to MM1 cells, the reaction was stopped after a certain time period, and then intracellular cAMP concentration was measured.

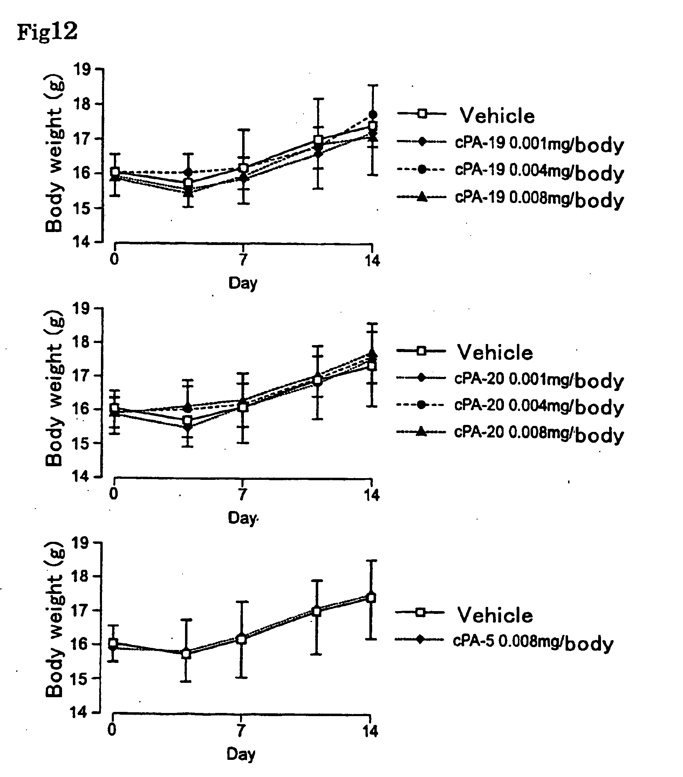

FIG 12 shows changes in body weight in each test group in an experiment wherein the suppressing actions of cPA-19 and cPA-20, the cyclic phosphatidic acid derivatives, on the lung metastasis of B16 melanoma, were examined by using a B16 melanoma hematogenous lung metastasis model.

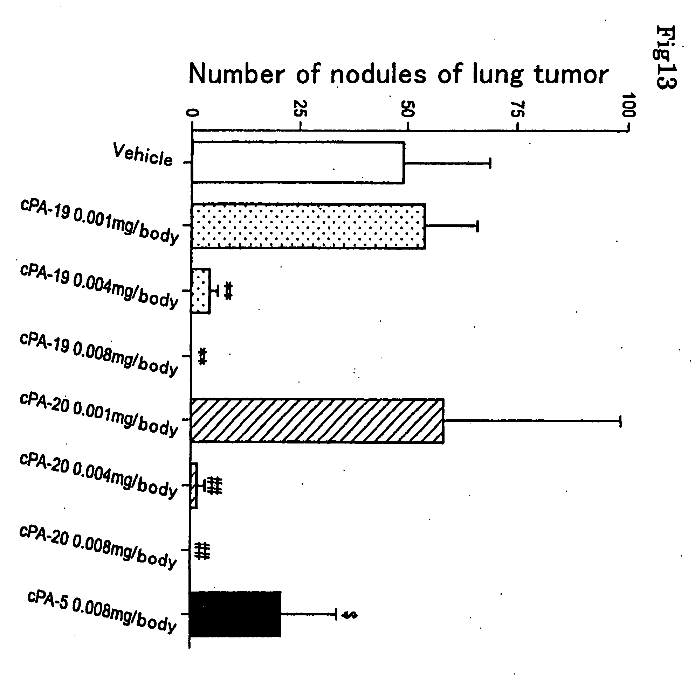

FIG. 13 shows the number of nodules formed as a result of lung metastasis in an experiment wherein the suppressing actions of cPA-19 and cPA-20, the cyclic phosphatidic acid derivatives, on the lung metastasis of B16 melanoma were examined by using the B16 melanoma hematogenous lung metastasis model.

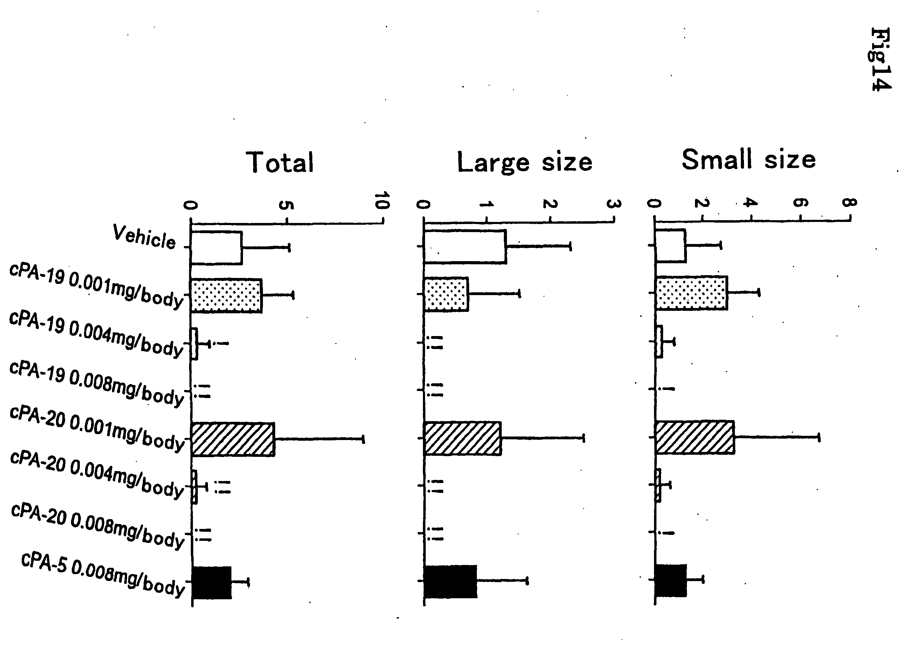

FIG. 14 shows the results of histopathological examination in an experiment wherein the suppressing actions of cPA-19 and cPA-20, the cyclic phosphatidic acid derivatives, on the lung metastasis of B16 melanoma were examined by using the B16 melanoma hematogenous lung metastasis model.

### BEST MODE OF CARRYING OUT THE INVENTION

Embodiments of the present invention will be hereinafter described in detail. General designations used in this specification are explained below.
- Ara-: arachic acid, 20:0
- BSA: bovine serum albumin
- Bt2cAMP: dibutyryl cyclic AMP
- cAMP: adenosine 3', 5'-cyclic phosphate
- CBM: carbamoyl
- cPA: cyclic phosphatidic acid
- CTX: cholera toxin
- DHA: docosahexaenoic acid
- EPA: eicosapentaenoic acid
- FBS: fetal bovine serum
- IBMX: 3-isobutyl-1-methylxanthine
- Lau-: lauric acid, 12:0
- LPA: lysophosphatidic acid
- MEM: minimum essential medium
- Ole-: oleic acid, 18:1
- Pal-: palmitic acid, 16:0
- ΔPal-: palmitoleic acid, 16:1
- PBS: phosphate buffered saline
- TCA: trichloroacetic acid

An agent for suppression of cancer metastasis according to the present invention contains a cyclic phosphatidic acid derivative represented by the formula (I) as an active ingredient: 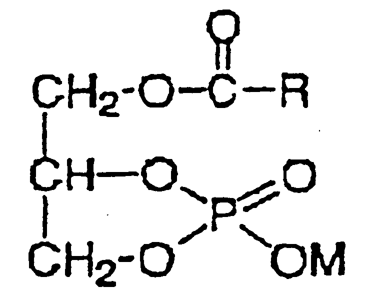 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

Examples of the C₁₋₃₀ linear or branched alkyl groups represented by the substituent R in the formula (I) include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an eicosyl group.

Examples of the C₂₋₃₀ linear of branched alkenyl group represented by the substituent R include an allyl group, a butenyl group, an octenyl group, a decenyl group, a dodecadienyl group, and a hexadecatrienyl group. More specifically, the examples include 8-decenyl group, 8-undecenyl group, 8-dodecenyl group, 8-tridecenyl group, 8-tetradecenyl group, 8-pentadecenyl group, 8-hexadecenyl group, 8-heptadecenyl group, 8-octadecenyl group, 8-icocenyl group, 8-dococenyl group, heptadeca-8,11-dienyl group, heptadeca-8,11,14-trienyl group, nonadeca-4,7,10,13-tetraenyl group, nonadeca-4,7,10,13,16-pentaenyl group, and henicosa-3,6,9,12,15,18-hexaenyl group.

The examples of the C₂₋₃₀ linear or branched alkynyl group represented by the substituent R include 8-decynyl group, 8-undecynyl group, 8-dodecynyl group, 8-tridecynyl group, 8-tetradecynyl group, 8-pentadecynyl group, 8-hexadecynyl group, 8-heptadecynyl group, 8-octadecynyl group, 8-icocynyl group, 8-dococynyl group, and heptadeca-8, 11-diynyl group.

The examples of the cycloalkane ring which may be contained in the above described alkyl group, alkenyl group or alkynyl group include, for example, a cyclopropane ring, a cyclobutane ring, a cyclopentane ring, a cyclohexane ring, and a cyclooctane ring. The cycloalkane ring may contain one or more hetero atoms, and examples thereof include an oxylane ring, an oxetane ring, a tetrahydrofuran ring, and an N-methylprolidine ring.

The examples of an aromatic ring which may be contained in the above described alkyl group, alkenyl group or alkynyl group include, for example, a benzene ring, a naphthalene ring, a pyridine ring, a furan ring, and a thiophene ring.

Accordingly, in the case where the substituent R is an alkyl group substituted with a cycloalkane ring, the examples include a cyclopropylmethyl group, a cyclohexylethyl group, and an 8,9-methanopentadecyl group.

In the case where the substituent R is an alkyl group substituted with an aromatic ring, the examples include a benzyl group, a phenetyl group, and a p-pentylphenyloctyl group.

M in the cyclic phosphatidic acid (cPA) derivative represented by the formula (I) is a hydrogen atom or a counter cation. In the case where M is a counter cation, examples thereof include an alkali metal atom, an alkali earth metal atom, and a substituted or unsubstituted ammonium group. The alkali metal atom includes, for example, lithium, sodium and potassium. The alkali earth metal atom includes, for example, magnesium and calcium. The substituted ammonium group includes, for example, a butylammonium group, a triethylammonium group and a tetramethylammonium group.

A compound represented by the formula (I) that is used as an active ingredient in the present invention can be synthesized according to methods described in publications (Kobayashi S et al., Tetrahedron Letters 34, 4047-4050 (1993); "The 23rd Symposium on Progression Organic Reactions in Life Science, November 17 and 18, 1997, The pharmaceutical society of Japan (Kumamoto civic center), Synthesis and physiological action of cyclic phosphatidic acid and carba derivative, Abstract collection pages 101-104."). An example of a specific synthetic pathway is shown in Fig. 6.

In Fig. 6, commercially available (R)-benzyl glycidyl ether (1) is activated with BF₃·Et₂O, and then a lithiated product, which is obtained by allowing n-BuLi to act on methylphosphonic acid dimethylester, is allowed to react with the activated product, thereby obtaining alcohol (2).

The obtained alcohol is allowed to react at 80°C using an excess of pyridinium salt of p-toluenesulfonic acid in toluene, thereby obtaining a cyclized product (3). The cyclized product is hydrolyzed using 20% Pd(OH)2-C under a hydrogen atmosphere, followed by debenzylation (4). 1-ethyl-3-(3-dimethylaminopropyl)carbodiimide hydrochloride is used as a condensing agent, and it is allowed to react with fatty acid, thereby obtaining a coupling product (5). Next, bromotrimethylsilane is used as a nucleophilic reagent, followed by position-selective removal of only methyl groups, thus cyclic phosphonic acid (6) is obtained. This product is transferred into a separatory funnel using ether, and then a small amount of 0.02 N sodium hydroxide aqueous solution is added dropwise to perform separation, and a compound of interest is extracted and purified as sodium salt (7).

The present invention relates to a novel agent for suppression of cancer metastasis.

Cancer metastasis comprises a multi-step process that begins with the detachment of cancer cells from the primary lesion, disruption of the extracellular matrix, invasion into blood vessels, invasion across the vascular endothelial cell layers, adhesion in the remote organ, and proliferation. Various factors are involved in these steps, and inhibitors of them are now attracting attention as a new agent for suppression of cancer metastasiss. Above all, invasion is the most characteristic step in the phenomenon of metastasis. It is considered that a superior anticancer agent is very likely to be developed from the substances that suppress the invasion of cancer cell. The present invention provides a novel agent for suppression of cancer metastasis which can be used as an agent for suppression of the invasion of cancer cell.

"Cancer metastasis" in the present invention means the shifting and proliferation of cancer cells from one part of the body to another organ, apparatus or the like. Specifically, it means that cancer cells shift to a site remote from the primary lesion so as to form cancer, and includes both hematogenous and lymphogenous metastasis.

The types of cancer whose metastasis should be suppressed by the agent for suppression of cancer metastasis of the present invention are not particularly limited, and include all forms of benign and malignant tumors. Specific examples of cancer whose metastasis should be suppressed include, but are not limited to, malignant melanoma, malignant lymphoma, digestive cancer, lung cancer, esophageal cancer, gastric cancer, large bowel cancer, rectal cancer, colon cancer, ureteral neoplasm, gallbladder cancer, bile duct cancer, biliary tract cancer, breast cancer, liver cancer, pancreatic cancer, testicular tumor, maxillary cancer, tongue cancer, lip cancer, oral cavity cancer, pharyngeal cancer, laryngeal cancer, ovarian cancer, uterine cancer, prostate cancer, thyroid cancer, brain tumor, Kaposi's sarcoma, hemangioma, leukemia, polycythemia vera, ganglioneuroblastoma, retinoblastoma, myeloma, bladder tumor, sarcoma, osteosarcoma, myosarcoma, skin cancer, basal cell carcinoma, skin appendage carcinoma, cutaneous metastatic carcinoma and skin malignant melanoma.

Organs to which cancer easily spreads by metastasis differ slightly depending on the type of cancer. For example, it is known that breast cancer spreads by metastasis at a higher rate to the lungs, liver, bone and lymph glands; gastric cancer to the liver, lungs, bone and adrenal gland; and large bowel cancer to the liver, lungs, adrenal glands and the like. Examples of metastatic cancer include metastatic liver cancer, metastatic jejunal carcinoma, metastatic bone tumor, metastatic brain tumor, metastatic lung tumor, cutaneous metastatic carcinoma, metastatic costal tumor, metastatic ovarian cancer and the like. Hence, the agent for suppression of cancer metastasis of the present invention can be used to suppress such cancer metastasis, and is particularly useful as an agent for suppression of postoperative metastasis. Moreover, the agent for suppression of cancer metastasis of the present invention is clinically an agent for suppression of recurrence of cancer, and is expected to prolong the survival period of patients and increase the survival rate.

The agent for suppression of cancer metastasis of the present invention can be used in combination with a known chemotherapy, a surgical treatment method, radiotherapy, hyperthermia, immunotherapy or the like. In particular, the effect of suppressing the cancer metastasis of the agent for suppression of cancer metastasis of the present invention can be further enhanced by combined use with a known anti-tumor agent and/or a substance that suppresses cancer metastasis. By the combined use with the agent for suppression of cancer metastasis of the present invention, the dose of a known anti-tumor agent and/or a cancer metastasis-suppressing substance to be administered can be reduced. Thus, side effects that are caused by these agents (e.g., leukopenia, thrombocytopia, bleeding, anaemia, anorexia, nausea, vomition, stomatitis, diarrhea, efflorescence, unhairing, pigmentation of skin, onset of fever, dullness, cephalalgia, liver functional impairment, proteinuria, edema and anaphylaxis) may be reduced.

The agent for suppression of cancer metastasis of the present invention is preferably provided in the form of a pharmaceutical composition which comprises one or more pharmaceutically acceptable additives and the compound of the formula (I) as an active ingredient.

The agent for suppression of cancer metastasis of the present invention can be administered in various forms, and the dosage forms may be peroral or parenteral (for examples, intravenous, intramuscular, subcutaneous or intracutaneous injection, rectal dosage, and permucosal dosage). Examples of the pharmaceutical composition suitable for peroral dosage include a tablet, a granule, a capsule, a powder, a solution, a suspension, and a syrup. Examples of the pharmaceutical composition suitable for parenteral dosage include an injection, an infusion, a suppository, and a percutaneous absorption agent. The dosage form of the medicament of the present invention is not limited to these. Furthermore, the agent of the present invention can also be made into sustained release formulations by publicly known methods.

The type of the pharmaceutical additives used for producing the agent of the present invention is not particularly limited, and can be suitably selected by a person skilled in the art. For examples, one can use an excipient, a disintegration agent or a disintegration auxiliary agent, a binder, a lubricant, a coating agent, a base, a solvent or a solubilizer, a dispersant, a suspension agent, an emulsifier, a buffer, an antioxidant, an antiseptic, an isotonic agent, a pH adjusting agent, a solving agent, and a stabilizer. Individual ingredients which are used for the above purposes are well known to a person skilled in the art.

Examples of the pharmaceutical additives usable for preparing a peroral preparation include an excipient such as glucose, lactose, D-mannitol, starch and crystalline cellulose; a disintegration agent or a disintegration auxiliary agent such as carboxymethyl cellulose, starch and carboxymethyl cellulose calcium; a binder such as hydroxypropyl cellulose, hydroxypropyl methylcellulose, polyvinyl pyrrolidone, and gelatin; a lubricant such as magnesium stearate and talc; a coating agent such as hydroxypropyl methylcellulose, white sugar, polyethylene glycol and titanium oxide; a base such as Vaseline, liquid paraffin, polyethylene glycol, gelatin, kaolin, glycerin, purified water, and hard fat.

Examples of the pharmaceutical additives which can be used for preparing an injection or an infusion preparation include a solvent or a solubilizer which can be used for an aqueous injection or a use-time dissolution type injection such as injection distilled water, physiological saline, and propylene glycol; an isotonic agent such as glucose, sodium chloride, D-mannitol, and glycerin; and a pH adjusting agent such as an inorganic acid, an organic acid, an inorganic base and an organic base.

The agent of the present invention can be administered to a mammal including human.

The dose of the agent of the present invention should be increased or decreased according to the conditions such as age, sex, body weight, symptom of a patient, and dosage route. The dose of the active ingredient per day for an adult is generally 1 pg/kg to 1,000mg/kg, and preferably 10µg/kg to 100mg/kg. The agent of the dose as mentioned above may be administered once a day, or may be dividedly administered a few times (for example, about 2-4 times) a day.

The present invention will be described in detail with reference to the following Examples, but the present invention is not limited by the Examples.

### EXAMPLE

### Example 1: Synthesis of cPA derivatives and suppression of cancer cell invasion thereby

### (1-1) Suppression of cancer cell invasion by various cPA synthetic derivatives

### (A) Materials and methods

### Chemical synthesis of cPA derivatives

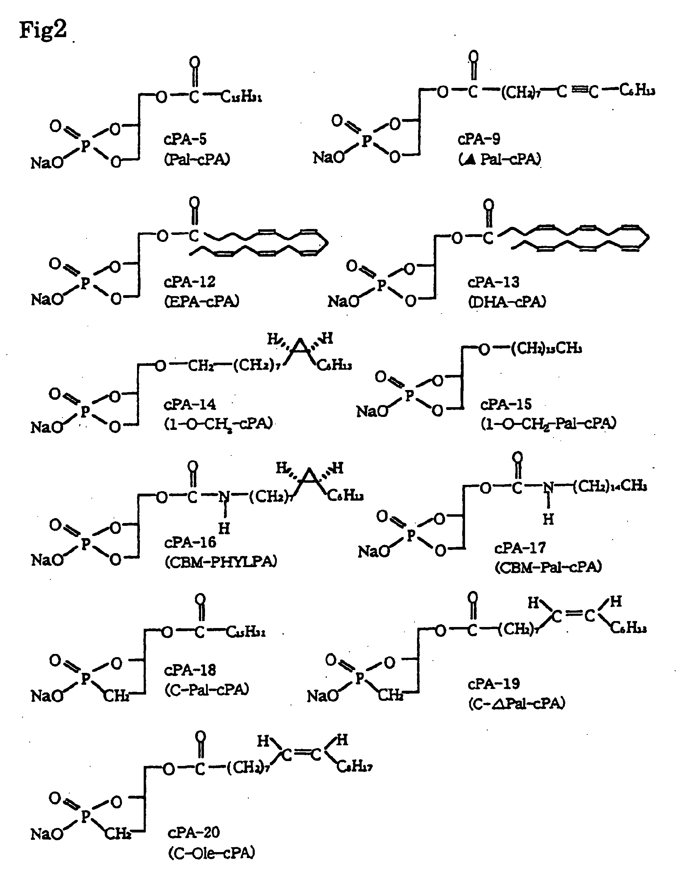

11 types of cPA synthetic derivatives were synthesized according to the method described by Kobayashi S et al., in Tetrahedron Letters 34, 4047-4050 (1993). Currently, 20 types of cPA derivatives have been synthesized. The invasion suppressing activity of cPA derivatives that were not used in this study has been already studied by Mukai et al. As a result, Pal-cPA and Ole-cPA suppressed LPA-induced invasion by approximately 85% and approximately 60%, respectively. PHYLPA, ΔPal-cPA, Lau-cPA and Ara-cPA suppressed LPA-induced invasion by around 50% (Mukai M et al., Int. J. Cancer 81, 918-922 (1999)). The structures of cPA derivatives used herein are shown in Fig. 2.

### Cell culture

The abdomen of a male DONRYU rat (300 g, NIPPON BIO-SUPP. CENTER) was opened aseptically. The thus obtained mesenterium was treated with 0.25% trypsin at 37°C for 15 minutes, filtrated with a 150 µm stainless mesh, and then centrifuged to collect mesothelial cells. The cells were cultured under conditions of 37°C and 5% CO₂ in a medium which was obtained by adding 10% FBS to Eagle's MEM1 (Nissui) supplemented with Eagle's MEM amino acid vitamin medium (Nissui) in a volume twice as great as that of the MEM1. A mesothelial cell monolayer which had reached a confluent density after 1 week of culturing was used for invasion assay.

The MM1 cells, which are highly invasive clones of rat ascites hepatoma cells (AH-130), were established by Mukai et al., (Int. J. Cancer : 81, 918-922 (1999)), and was cultured under the same conditions as those for mesothelial cells.

### In vitro invasion assay

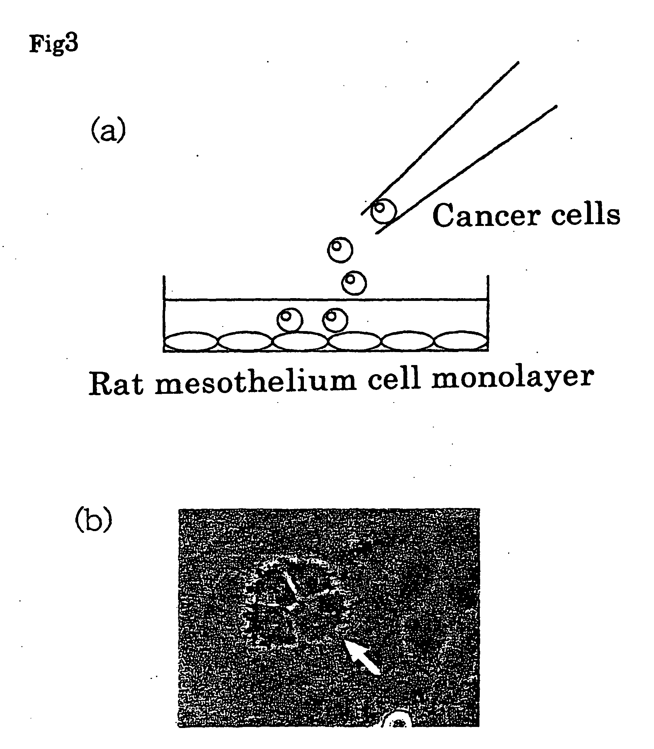

In vitro invasion assay was developed by Akedo et al (Akedo, H et al., Cancer Res. 46, 2416-2422 (1986)). MM1 cells were multi-layer-cultured on the mesothelial cell layer, which had been grown to reach a confluent density, in the presence of 10% FBS or 25 µM LPA (SIGMA). 25 µM cPA was then added. The cells were cultured under conditions of 37°C and 5% CO₂ for 20 hours, and then fixed with 10% formalin. The number of colonies of MM1 cells that had invaded was counted under a phase-contrast microscope (Fig. 3).

cPA and LPA were ice-cooled in PBS containing 0.1% fatty-acid free BSA (SIGMA), ultrasonicated, emulsified, stored at -20°C, and then used.

### (B) Results and discussion

### Suppression of cancer cell invasion by 11 types of cPA derivatives

Of some cPA derivatives, Pal-cPA was shown to also strongly suppress both FBS-induced invasion and LPA-induced invasion (Mukai, M. et al., Int. J. Cancer 81, 918-922 (1999)). In order to examine the relationship between cPA structure and the intensity of invasion suppression, the effects of, 11 types of cPA derivatives including Pal-cPA which were chemically synthesized, on cancer cell invasion, were examined.

The cells were fixed 20 hours after the start of invasion assay in the presence of 10% FBS, and then the number of colonies that had invaded was counted. Thus, for 5 types of 25 µM cPA derivatives other than Pal-cPA, invasion suppression of more than 50% was observed (Fig. 4). For these 5 types, the effect on LPA-induced invasion was further examined (Fig. 5). As a result, differences were observed in terms of invasion suppressing activity depending on the differences in the type of fatty acid that binds at the position 1 of the glycerol backbone. FBS-induced invasion was suppressed by approximately 70% by cPA-12 having EPA at the sn-1 position, but LPA-induced invasion was not suppressed. Accordingly, it is considered that FBS may contain an invasion-promoting factor other than LPA, and cPA-12 may suppress invasion which is induced by this factor. This also agrees with the fact that invasion of MM1 cells is induced by 10% FBS or 25 µM LPA to the same degree, but LPA concentration in a medium containing 10% FBS is approximately a tenth part thereof. Furthermore, it was shown that the specificity of invasion suppressing action by cPA was provided by the fatty acid types. Further examination is required for the structure-activity correlation. Moreover, strong invasion suppressing activity was observed in carba-cPA that had been designed to prevent ring-opening of cyclic phosphate groups such that O at the sn-3 position had been substituted with C.

Furthermore, even when only MM1 cells were pre-treated with cPA, washed with a medium, and then overlaid on mesothelial cells in the presence of LPA, the invasion suppressing activity was not lost. When only mesothelial cells were pre-treated with cPA, washed, and then overlaid with MM1 cells in the presence of LPA, clear invasion was observed. Taken together, it was shown that cPA has its effect not on mesothelial cells but on MM1 cells.

### (1-2) Suppression of cancer cell invasion by Carba-cPA

### (A) Materials and methods

### Synthesis of Carba-cPA

The synthesis method of Carba-cPA has been developed by Susumu KOBAYASHI, Faculty of Pharmaceutical Sciences, Science University of Tokyo. Numbers in the following explanation correspond to the numbers in Fig. 6.

Commercially available (R)-benzyl glycidyl ether (1) is activated with BF₃·Et₂O, and then a lithiated product, which is obtained by allowing n-BuLi to act on methylphosphonic acid dimethylester, is allowed to react with the activated product, thereby obtaining alcohol (2). The obtained alcohol is allowed to react at 80°C using an excess of pyridinium salt of p-toluenesulfonic acid in toluene, thereby obtaining a cyclized product (3). The cyclized product was hydrolyzed using 20% Pd(OH)₂-C under a hydrogen atmosphere, followed by debenzylation (4). 1-ethyl-3-(3-dimethylaminopropyl)carbodiimide hydrochloride was used as a condensing agent, and it was allowed to react with fatty acid, thereby obtaining a coupling product (5). Next, bromotrimethylsilane was used as a nucleophilic reagent, followed by position-selective removal of only methyl groups, so that cyclic phosphonic acid (6) was obtained. This product was transferred into a separatory funnel using ether, and then a small amount of 0.02 N sodium hydroxide aqueous solution was added dropwise to perform separation. Then, a compound of interest was extracted and purified as sodium salt (7).

### In vitro invasion assay using carba-cPA at low concentration

0.5µM, 1µM and 10µM carba-cPA were added to MM1 cells, and then in vitro invasion assay was performed similarly to (1-1) in the presence of 25µM LPA

### Effect of carba-cPA on the proliferation of MM1 cells

12.5µM and 25µM cPA were added to media containing 10% FBS, and then the cells were cultured under conditions of 37°C and 5% CO₂ for 24 hours and 72 hours. The number of the MM1 cells was counted using a hemocytometer.

### (B) Results and discussion

### Suppression of cancer cell invasion by carba-cPA with low concentration

To examine the differences in suppressing activity resulting from different fatty acid types, carba-cPA at a low concentration was added, and then invasion assay was performed (Fig. 7).

Among the natural cPA, one having palmitic acid bound at the sn-1 position most strongly suppressed the invasion. Among the carba-cPA, cPA-19 having oleic acid bound thereto and cPA-20 having palmitoleic acid bound thereto exhibited a stronger invasion suppressing activity than cPA-18 having palmitic acid bound thereto.

### Effect of carba-cPA on MM1 cell proliferation

As shown in Fig. 8, cPA having palmitic acid bound at the sn-1 position, that is, both natural cPA (cPA-5) and carba-derivative (cPA-18) had no effect on MM1 cell proliferation. Furthermore, carba-Ole-cPA (cPA-19) and carba-ΔPal-cPA (cPA-20) that exhibited even stronger invasion suppressing activity also showed proliferation suppressing activity. Even forms of natural cPA, that is, both Ole-cPA and ΔPal-cPA, were shown to have proliferation suppressing activity. However, Mukai et al have reported that the invasion suppressing action of these molecular types is weak (Mukai, M., et al., Int. J. Cancer 81, 918-922 (1999)). Therefore, it is considered that the effects of different fatty acid types on cell proliferation may be a phenomenon independent from invasion suppressing action.

### Example 2: Effect of cPA on serum/LPA-independent cancer cell invasion

### (A) Materials and methods

### Cell culture

HT-1080 cell which is human fibrosarcoma cell was established by Mukai et al (Int. J. Cancer: 81, 918-922 (1999)). HT-1080 cells were cultured under conditions of 37°C and 5% CO₂ in Eagle's MEM1 (Nissui) supplemented with a one-hundredth volume of nonessential amino acid solution (GIBCO BRL) and 10% FBS.

### In vitro invasion assay

HT-1080 cells were collected by 0.05% trypsin treatment, washed with a serum-free medium, and then subjected to assay similar to that shown in Chapter 1 in the absence of serum/LPA. 4 hours later, the cells were fixed, and then the cells that had invaded were counted.

### In vitro invasion assay of MM1 cells using the culture supernatant of HT-1080 cells

The medium of HT-1080 cells was exchanged with a serum-free medium, and then the cells were cultured under conditions of 37°C and 5% CO₂ for 20 hours. The medium was collected and centrifuged. The resulting supernatant was used as a culture supernatant. MM1 cells that had been washed once were suspended in this supernatant, and then invasion assay was performed similarly to that described in paragraph 0043.

### (B) Results and discussion

### Effect of carba-cPA on serum/LPA-independent cancer cell invasion

In the in vitro invasion assay system, HT-1080 cells invade in a Serum/LPA-independent manner. This phenomenon has also been shown to be suppressed by Pal-cPA (Mukai, M., et al., Int. J. Cancer 81, 918-922 (1999)). Based on this finding, the effect of carba-cPA on the invasion of HT-1080 cells was examined using cPA-18 (carba-Pal-cPA) having the same fatty acid. As shown in Fig. 9, carba-cPA also exhibited invasion suppressing activity on the cells at the same strength as or a level stronger than that exhibited by natural cPA.

### Effect of the culture supernatant of HT-1080 cells on MM1 cell invasion

HT-1080 cells may synthesize LPA themselves, and the invasion may be induced by the LPA. In consideration of this possibility, MM1 cells that invade LPA-dependently were suspended in the culture supernatant of HT-1080 cells, and then overlaid on mesothelial cells, and the occurrence of the invasion was examined. MM1 cells (1.5x10⁵cells/ml) were suspended in the serum-free culture supernatant of HT-1080 cells, and then subjected to in vitro invasion assay. The obtained results are shown in Table 1.

As a result, MM1 cell invasion was not promoted (Table 1). Moreover, even when 25 µM LPA was added to the in vitro invasion assay system using HT-1080 cells, the invasion was no longer promoted. These results suggest that the invasion-promoting factor of HT-1080 cells is different from LPA.

**Table 1**

| Medium added | Number of invasion cells/cm² |
|---|---|
| Untreated medium | 97.7±38.4 |
| Conditioned medium | 72.2±18.4 |

### Example 3: Analysis of signal transduction system for the suppression of invasion by cPA

### (A) Materials and methods

### Effect of a reagent for increasing intracellular cAMP concentration on invasion

The following reagents were respectively added to an in vitro invasion assay system so as to examine their effects on MM1 cell invasion in the presence of 25 µM LPA: Forskolin (Wako) which directly activates adenylate cyclase (cAMP synthase); cholera toxin (CTX) (GIBCO BRL) which prevent the deactivation of G protein (Gs) which activates adenylate cyclase; Bt₂cAMP (nacalai tesque) which can pass through the membrane and functions in a manner similar to that of cAMP when it enters the cell; or isobutylmethylxanthine (IBMX, 3-isobutyl-1-methylxanthine) (SIGMA) which inhibits phosphodiesterase (cAMP degrading enzyme).

### Measurement of intracellular cAMP

The MM1 cells in a serum-free medium were pretreated with 1 mM IBMX for 10 minutes, and then, 25 µM LPA or cPA, or 10 µM Forskolin as a positive control which had been confirmed to suppress. the invasion, was added. After a certain time period, 10% ice-cooled TCA was added to stop the reaction. The centrifuged supernatant was washed 4 times with water-saturated diethylether to remove TCA, and then placed under nitrogen gas flow at 60°C to remove the solvent completely. cAMP in the resultant sample was measured using a cyclic AMP [³H] assay system (Amersham).

### (B) Results and discussion

### Effect of elevated intracellular cAMP concentration on cancer cell invasion

It is known that elevated intracellular cAMP concentration inhibits the activity of Rho, which is one of low-molecular-weight G proteins, through activation of cAMP-dependent protein kinase A (Laudanna, C., et al., J. Biol. Chem. 272, 24141-24144 (1997) and Dong, J-M., et al., J. Biol. Chem. 273, 22554-22562 (1998)). In addition, it has been reported that Pal-cPA leads an increase of intracellular cAMP concentration of MM1 cells (Mukai, M., et al., Int. J. Cancer 81, 918-922 (1999)). Accordingly, the effect of cAMP on LPA-induced invasion that has been shown to be mediated by Rho was examined.

As shown in Fig. 10, any agent suppressed the cancer cell invasion. Accordingly, using Forskolin, one of these reagents, as a positive control, the changes in intracellular cAMP concentration resulting from the addition of carba-cPA were measured, and the involvement in the invasion suppressing activity was examined.

### Effect of Carba-cPA on intracellular cAMP concentration

As shown in Fig. 11, although an increase of cAMP concentration resulting from the presence of Forskolin could be measured, a previously reported rapid increase of cAMP concentration resulting from the addition of Pal-cPA could not be confirmed. Even when Carba-Pal-cPA was added, a similar result was obtained. However, the invasion of the same cells was induced by LPA, and the invasion was suppressed by Pal-cPA, suggesting the presence of another invasion suppressing mechanism that is not mediated by cAMP.

### (Conclusion of Examples 1 to 3)

In Example 1, in vitro invasion assay was performed using various cPA derivatives. First, when the effect of cPA on serum-induced invasion was examined, differences in invasion suppressing activity were observed because of structural difference, as shown in Fig. 4. Of these derivatives, 6 types of cPA derivatives including Pal-cPA, which exhibit the invasion suppressing activity of more than 50% when added at 25 µM, were examined for their effects on LPA-induced invasion (Fig. 5). cPA-12 having EPA at the sn-1 position, which had suppressed the serum-induced invasion by approximately 70%, did not suppressed the LPA-induced invasion. This result suggested the possibility of the presence of an invasion-promoting substance other than LPA in the serum. Furthermore, invasion suppressing activity was found in carba-cPA that had been designed to prevent ring-opening of cyclic phosphate groups such that O at the sn-3 position had been substituted with C. This activity was significantly strong to the extent that it was found to be 10 to 100 times greater than that exhibited by natural cPA (Fig. 7).

The carba-cPA was focused in Example 2. The effect of carba-cPA on the invasion of cancer cells that do not require serum/LPA for their invasion was examined. As shown in Fig. 9, carba-cPA was observed to exhibit strong invasion suppressing activity on LPA-independent invasion as in the case of natural cPA.

Furthermore, the action and mechanism of invasion suppression by carba-cPA were analyzed in Example 3. LPA promotes the invasion through the activation of Rho, which is one of low-molecular-weight proteins. Moreover, intracellular cAMP concentration is elevated by the addition of Pal-cPA so that Rho activity is suppressed. In consideration of the thus assumed possibility that carba-cPA may also cause a rise in inracellular cAMP concentration so as to suppress the invasion, intracellular cAMP concentration was measured (Fig. 11). However, although an elevated cAMP concentration was measured by Forskolin which is a positive control, the rise in cAMP concentration resulting from Pal-cPA could not be confirmed. Even in the case of carba-Pal-cPA, the change of cAMP concentration was not observed. The fact that no reproducibility was observed for Pal-cPA may be due to a change in MM1 cells. However, the serum/LPA-induced invasion and the invasion suppression by Pal-cPA were also observed in this cell, suggesting the presence of another invasion-suppressing system that is not mediated by cAMP.

### Example 4: Suppressing action of cyclic phosphatidic acid derivatives cPA-19 and cPA-20, on the lung metastasis of B16 melanoma using B16 melanoma hematogenous lung metastasis model

### (Test materials and methods)

### 1. Test substance, medium and positive control substance

cPA-19 and cPA-20 were used as test substances. 0.1 w/v% BSA/PBS which was prepared by dissolving Albumin, Bovine [essentially Fatty Acid Free] (BSA, Lot No. 89H7604, Sigma chemical company) in Ca²⁺ and Mg²⁺ free PBS (pH 7.2), was used as a medium After preparation, the solution was sterilized by filtration using a filter (0.22 µm, Millipore). Preparation was performed when used. cPA-5 was used as a positive control substance.

### 2. Preparation method for mixture and frequency

A medium was added in a volume required for a test substance or a positive control substance. Dissolution was performed by ultrasonication, thereby preparing a 0.16 mg/mL solution. The 0.16 mg/mL solution containing a test substance was diluted with a medium, thereby preparing 0.08 and 0.02 mg/mL solutions. All mixtures were prepared when used.

### 3. Test system and environmental conditions therefor

Mice were purchased from CHARLES RIVER JAPAN. INC. The line employed herein was C57BL/6NCrj (sexuality: female). The number of animals kept in stock was 60, and the age of these animals when kept in stock was 4 weeks. The body weight ranged from 10.2 to 14.0 g. The environmental conditions for the test system were as follows:
Temperature: 23 to 26°C
Humidity: 54 to 60°C
Ventilation frequency: 13 times/hour
Lighting: 12 hours (from 7:00 a.m. to 7:00 p.m.)
Feed: Cubed diet CRF-1 (Oriental Yeast Co., Ltd.) subjected to high pressure steam sterilization, was given adlib.
Drinking water: Well water supplemented with sodium hypochlorite (residual chlorine concentration: approximately 2 ppm) was given adlib by a water supply pot.
Bedding: White flakes (CHARLES RIVER JAPAN. INC.) subjected to high pressure steam sterilization were used.
Breeding cage and exchange frequency: Cages made by polycarbonate subjected to high pressure steam sterilization were used (W215×H140×D320 mm). Cages were exchanged twice a week simultaneously with the exchange of bedding materials.
Number of animals housed in a cage: During the quarantine and acclimatization period, 5 mice were housed per cage. During the test period, 3 mice were housed per cage.
Washing and sterilization: The breeding room was cleaned every day, and the floor was cleansed with an antiseptic solution.

### 4. Test method

### 4.1 Name of tumor cells

Mouse malignant melanoma B16-F0 was used as a tumor cell. The cell was purchased from Dainippon Pharmaceutical Co., Ltd.

### 4.2 Culture

Dulbecco's Modified Eagle's Media containing 10% feral bovine serum and kanamycin were used for culture. The cells were static-cultured in a CO₂ incubator set at 37°C and 5% CO₂ under conditions of 95% humidity. After the tumor cells reached a semi-confluent state, the cells were harvested using a 0.25% trypsin-0.02% EDTA mixture. A medium was added to stop the action of trypsin while pipetting, thereby preparing a cell suspension. The suspension was diluted by one-tenth with a medium for culturing, followed by subculturing until it reached a volume sufficient for grafting.

### 4.3 Preparation of tumor cell suspension for grafting

A cell suspension was prepared by the same technique as the subculture method. Specifically, the cells were washed with a Ca²⁺ and Mg²⁺ free phosphate buffer [PBS(-)], and then harvested. The harvested cells were suspended in a 10 mL of PBS(-), thereby preparing a cell suspension. The number of cells in the cell suspension was measured to obtain the total cell number. 50 µL of the cell suspension, 100 µL of 0.4% trypan blue liquid and 100 µL of PBS(-) were mixed, and then viable count was measured. The survival rate of the cells was calculated from the viable count and total cell number. Based on these results, the viable count was adjusted at 1x10⁷ cells/mL, thereby preparing a tumor cell suspension for grafting.

### 4.4 Administration

Based on the body weight on the day of the administration of the test substance, mice were grouped into 8 test groups according to the stratified continuous randomization method as shown in Table 2 below.

**Table 2**

| Test group | Dose (mg/body) | Concentration of substance administered (mg/0.05mL) | Tumor cell suspension (cells/0.05mL) | Dose volume (mL/body) | Route of administration | Number of animals used |
|---|---|---|---|---|---|---|
| Medium | --- | --- | 5×10⁵ | 0.1 | i.v. | 6 |
| cPA-19 | 0.001 | 0.001 | | | | 6 |
| | 1004 | 1004 | | | | 6 |
| | 0.008 | 0.008 | | | | 6 |
| cPA-20 | 0.001 | 0.001 | | | | 6 |
| | 0.004 | 0.004 | | | | 6 |
| | 0.008 | 0.008 | | | | 6 |
| CPA-5 | 0.008 | 0.008 | | | | 6 |

Previous test results revealed that neither test substance nor positive control substance exert proliferation suppression action on B16 mouse malignant melanoma. Hence, the medium, the test substance or the positive control substance was mixed with B16-F0 cell suspension at a ratio of 1:1. The mixture was administered via the caudal vein. The number of grafted cells was 5x10⁵ cells/body. The dose of the test substance was 0.001, 0.004 or 0.008 mg/body, and the dose of the positive control substance was 0.008 mg/body.

### 4.5 Observation of general state and measurement of body weight

Observation period ranged from days 0 to 14 after the administration of the test substance. Life and death were confirmed every day during this period, and the general state was observed. Body weight measurement was performed twice a week.

### 4.6 Autopsy

On day 14 after the administration of the test substance, blood was collected from the posterior portion of the abdominal vena cava under isoflurane anesthesia. After the mice were sacrificed by blood-letting, the presence or absence of intrapleural and intraperitoneal metastatic nodules was confirmed. Lungs were extracted, and then fixed with a 10% neutral buffered formalin solution. The number of nodules resulting from lung metastasis was measured under a stereoscopic microscope (magnification: 10 times).

### 4.7 Histopathological examination

After the measurement of the number of nodules resulting from lung metastasis, samples were paraffin-embedded, and then thin sections were prepared. Hematoxylin eosinic-stained samples were prepared from these sections. The number of metastatic lesions in the certain portions of the left lobe and the posterior lobe was measured, and then the lesions were sorted by size. On the basis of the diameter (0.65 mm) of an object lens (x40), metastatic lesions with major diameters of less than 0.65 mm were classified as having a small size, and those with major diameters of between 0.65 mm or more and less than 1.30 mm were classified as having a large size. Furthermore, the degree of invasion of lymphocytes into metastatic lesions was confirmed qualitatively.

### 5. Statistical analysis method

The representative value of each test group was denoted as mean ± standard deviation (mean ± S.D.). Differences in mean values were examined as follows. The number of metastatic nodules was examined by Dunnett's multiple comparison test between the medium group and the cPA-19 group, and between the medium group and the cPA-20 group. The same was examined by the Student's T-test between the medium group and cPA-5 group. The number of metastatic lesions was examined by the Wilcoxon test using the medium group as a control. The number of animals having metastatic nodules or metastatic lesions was examined by Fisher's extract probability test using the medium group as a control. The significance levels were 1% and 5%.

### (Results)

### 1. General state

In all the test groups, no deaths were observed during the observation period. In addition, no abnormalities were observed in the general state.

### 2. Changes in body weight (Fig. 12 and Table 3)

Changes in body weight in each test group are shown in Fig. 12 and Table 3.

**Table 3: Effect of cPA-19 or cPA-20 on body weight of B16-F0 melanoma-bearing mouse**

| Group | Dose (mg/body) | N | Body weight (g) | | | | |
|---|---|---|---|---|---|---|---|
| | | | day 0 | day 4 | day 7 | day 11 | day 14 |
| Solvent | --- | 6 | 16.0±0.5 | 15.7±0.8 | 16.1±1.1 | 16.9±1.2 | 17.3±1.2 |
| | | | | | | | |
| cPA-19 | 0.001 | 6 | 15.9±0.6 | 15.5±0.5 | 15.8±0.7 | 16.5±1.0 | 17.1±1.2 |
| | 0.004 | 6 | 15.9±0.6 | 16.0±0.5 | 16.1±0.6 | 16.7±0.6 | 17.6±0.9 |
| | 0.008 | 6 | 15.0±0.6 | 15.4±0.5 | 15.9±0.5 | 16.8±0.5 | 17.0±0.4 |
| | | | | | | | |
| cPA-20 | 0.001 | 6 | 15.9±0.6 | 15.5±0.3 | 16.1±0.6 | 16.8±0.4 | 17.4±0.6 |
| | 0.004 | 6 | 16.0±0.6 | 16.0±0.7 | 16.2±0.6 | 16.9±0.7 | 17.5±0.8 |
| | 0.008 | 6 | 15.9±0.5 | 16.1±0.8 | 16.3±0.8 | 17.0±0.9 | 17.7±0.8 |
| | | | | | | | |
| CPA-5 | 0.008 | 6 | 15.9±0.6 | 15.8±0.9 | 16.2±1.0 | 17.0±0.8 | 17.4±1.0 |

| | | | | | | | |
|---|---|---|---|---|---|---|---|
| Each value denotes mean ± S.D. There was no significant difference from the solvent group. | | | | | | | |

On the day of the administration of the test substance, the mean body weight in the medium group was 16.0±0.5 g. The mean body weights in the groups administered with cPA-19 at 0.001, 0.004 and 0.008 mg/body were 15.9±0.6, 15.9±0.6 and 15.9±0.6 g, respectively. The mean body weights in the groups administered with cPA-20 at 0.001, 0.004 and 0.008 mg/body were 15.9±0.6, 16.0±0.6 and 15.9±0.5g, respectively. The mean body weight in the cPA-5 group was 15.9±0.6 g. On day 4 after the administration of the test substance, the mean body weight in the medium group was 15.7±0.8 g. The mean body weights in the groups administered with cPA-19 at 0.001, 0.004 and 0.008 mg/body were 15.5±0.5, 16.0±0.5 and 15.4±0.5 g, respectively. The mean body weights in the groups administered with cPA-20 at 0.001, 0.004 and 0.008 mg/body were 15.5±0.3, 16.0±0.7 and 16.1±0.8 g, respectively. The mean body weight in the cPA-5 group was 15.8±0.9 g. On day 7 after the administration of the test substance, the mean body weight in the medium group was 16.1±1.1 g. The mean body weights in the groups administered with cPA-19 at 0.001, 0.004 and 0.008 mg/body were 15.8±0.7, 16.1±0.6 and 15.9±0.5 g, respectively. The mean body weights in the groups administered with cPA-20 at 0.001, 0.004 and 0.008 mg/body were 16.1±0.6, 16.2±0.6 and 16.3±0.8 g, respectively. The mean body weight in the cPA-5 group was 16.2±1.0 g. On day 11 after the administration of the test substance, the mean body weight in the medium group was 16.9±1.2 g. The mean body weights in the groups administered with cPA-19 at 0.001, 0.004 and 0.008 mg/body were 16.5±1.0, 16.7±0.6 and 16.8±0.5 g, respectively. The mean body weights in the groups administered with cPA-20 at 0.001, 0.004 and 0.008 mg/body were 16.8±0.4, 16.9±0.7 and 17.0±0.9 g, respectively. The mean body weight in the cPA-5 group was 17.0±0.8 g. On day 14 after the administration of the test substance, the mean body weight in the medium group was 17.3±1.2 g. The mean body weights in the groups administered with cPA-19 at 0.001, 0.004 and 0.008 mg/body were 17.1±1.2, 17.6±0.9 and 17.0±0.4 g, respectively. The mean body weights in the groups administered with cPA-20 at 0.001, 0.004 and 0.008 mg/body were 17.4±0.6, 17.5±0.8 and 17.7±0.8 g, respectively. The mean body weight in the cPA-5 group was 17.4±1.0 g. At the time of each measurement, the body weight value of each group was compared with that of the medium group. Thus, no significant difference was found between cPA-19, cPA-20 or cPA-5 group with the medium group. Furthermore, the body weight value at each measurement time was compared with that on the day of the administration of the test substance. Thus, on day 4 after the administration of the test substance, temporal decreases in body weight were observed in the medium group, the groups administered with cPA-19 at 0.001 and 0.008 mg/body, the group administrated with cPA-20 at 0.001 mg/body, and the cPA-5 group.

### 3. Nodules resulting from lung metastasis (Fig. 13 and Table 4)

The results of examining the number of metastatic nodules in the lungs (extracted on day 14 after the administration of the test substance) are shown in Fig. 13 and Table 4.

**Table 4: Effect of cPA-19 or cPA-20 on lung metastasis of B16-F0 melanoma in mouse**

| Group | Dose (mg/body) | N | Number of mice having lung tumor nodules | Number of lung tumor nodules | Range (mininum-maximum) |
|---|---|---|---|---|---|
| Solvent | --- | 6 | 6 | 49±20 | 28-84 |
| | | | | | |
| cPA-19 | 0.001 | 6 | 6 | 54±12 | 37-66 |
| | 0.004 | 6 | 6 | 4±2** | 2-6 |
| | 0.008 | 6 | 0 && | 0±0** | 0 |
| | | | | | |
| cPA-20 | 0.001 | 6 | 6 | 58±40 | 17-112 |
| | 0.004 | 6 | 2 & | 1±2## | 0-4 |
| | 0.008 | 6 | 1 && | 0±0## | 0-1 |
| | | | | | |
| CPA-5 | 0.008 | 6 | 6 | 21±13$ | 5-38 |

| | | | | | |
|---|---|---|---|---|---|
| &P<0.05, &&P<0.01 vs. solvent group (Fisher's extract probability test) **P<0.01 vs. solvent group (Dunnett's multiple comparison test) ##P<0.01 vs. solvent group (Dunnett's multiple comparison test) $P<0.05 vs. solvent group (Student's T test). | | | | | |

The number of mice having metastatic nodules was 6 in the medium group. 6, 6 and 0 mice in the groups administered with cPA-19 at 0.001, 0.004 and 0.008 mg/body, respectively, had metastatic nodules. 6, 2 and 1 mice in the groups administered with cPA-20 at 0.001, 0.004 and 0.008 mg/body, respectively, had metastatic nodules. 6 mice in the cPA-5 group had metastatic nodules. Compared with the medium group, the number of mice having metastatic nodules decreased significantly in the groups administered with cPA-19 at 0.008 mg/body, and cPA-20 at 0.004 and 0.008 mg/body.

The number of metastatic nodules in the medium group was 49±20 (minimum-maximum: 28-84; the same applies to the following explanation). In the groups administered with cPA-19 at 0.001, 0.004 and 0.008 mg/body, 54±12(37-66), 4±2(2-6) and 0±0 (0) nodules were respectively present. In the groups administered with cPA-20 at 0.001, 0.004 and 0.008 mg/body, 58±40(17-112), 1±2(0-4) and 0±0(0-1) nodules, respectively, were present. In the cPA-5 group, 21±13(5-38) nodules were present. Compared with the medium group, the number of nodules resulting from lung metastasis significantly decreased in the groups administered with cPA-19 at 0.004 and 0.008 mg/body and cPA-20 at 0.004 and 0.008 mg/body, and in the cPA-5 group.

### 4. Autopsy (Table 5)

The results of autopsy are shown in Table 5.

**Table 5: Effect of cPA-19 or cPA-20 on intrapleural and intraperitoneal metastatic nodules**

| Abdominal cavity | | | | | | | | | | |
|---|---|---|---|---|---|---|---|---|---|---|
| Group | Dose (mg/ body) | Animal No. | Thoracic cavity | Diaphragm | Spleen | Adrenal gland | Kidney | Ovary | Adipose tissue | Tissue around glandual clitoris |
| Solvent | - | 301 | - | - | + | - | - | + | + | - |
| | | 302 | - | - | + | - | - | - | - | - |
| | | 303 | - | - | - | - | - | + | - | - |
| | | 304 | - | - | + | - | - | - | - | - |
| | | 305 | - | - | + | - | - | - | + | - |
| | | 306 | + | - | + | - | - | - | - | - |
| | 0.001 | 307 | - | - | - | + | + | + | + | - |
| | | 308 | - | - | - | - | + | + | - | - |
| | | 309 | + | - | + | - | - | - | - | - |
| | | 310 | - | - | - | - | - | - | - | - |
| | | 311 | - | - | - | - | - | - | - | - |
| | | 312 | - | - | - | - | - | - | - | - |
| cPA-19 | 0.004 | 313 | - | - | + | - | - | - | - | - |
| | | 314 | - | - | - | - | - | - | - | - |
| | | 315 | - | - | - | - | - | - | - | - |
| | | 316 | - | - | - | - | - | - | - | - |
| | | 317 | - | - | - | - | - | - | - | - |
| | | 318 | - | - | - | + | - | - | + | - |
| | 0.008 | 319 | - | - | - | - | - | - | - | - |
| | | 320 | - | - | - | - | - | - | - | - |
| | | 321 | - | - | - | - | - | - | - | - |
| | | 322 | - | - | - | - | - | - | - | - |
| | | 323 | - | - | + | - | - | - | - | - |
| | | 324 | - | - | - | - | - | - | - | - |
| cPA-20 | 0.001 | 325 | - | - | - | - | - | - | - | - |
| | | 326 | - | - | + | - | - | - | - | - |
| | | 327 | + | + | - | - | - | - | - | + |
| | | 328 | - | - | - | - | - | + | - | - |
| | | 329 | - | - | - | - | - | - | - | - |
| | | 330 | - | - | - | - | - | - | - | - |
| | 0.004 | 331 | - | - | - | - | - | - | - | - |
| | | 332 | - | - | - | - | - | - | - | - |
| | | 333 | - | - | - | - | - | - | - | - |
| | | 334 | - | - | - | - | - | - | - | - |
| | | 335 | - | - | - | - | - | - | - | - |
| | | 336 | - | - | + | - | - | - | - | - |
| | 0.008 | 337 | - | - | - | - | - | - | - | - |
| | | 338 | - | - | - | - | - | - | - | - |
| | | 339 | - | - | - | - | - | - | - | - |
| | | 340 | - | - | - | - | - | - | - | - |
| | | 341 | - | - | - | - | - | - | - | - |
| | | 342 | - | - | + | - | - | - | - | - |
| cPA-5 | 0.008 | 343 | - | - | + | - | - | - | - | - |
| | | 344 | - | - | + | - | - | - | - | - |
| | | 345 | - | - | + | - | - | - | - | - |
| | | 346 | - | - | - | - | - | - | - | - |
| | | 347 | - | - | - | - | - | - | - | - |
| | | 348 | - | - | - | - | - | - | - | - |

| | | | | | | | | | | |
|---|---|---|---|---|---|---|---|---|---|---|
| - : Absent + : Present | | | | | | | | | | |

In the medium group, metastatic nodules were observed in the thoracic cavity (1 case), and in the abdominal cavity: the spleen (5 cases), ovary (2 cases) and adipose tissue (2 cases). In the group administered with cPA-19 at 0.001 mg/body, metastatic nodules were observed in the thoracic cavity (1 case) and in the abdominal cavity: the spleen (1 case), ovary (2 cases), adrenal gland (1 case), kidney (2 cases) and adipose tissue (1 case). In the group administered with cPA-19 at 0.004 mg/body, no metastatic nodules were observed in the thoracic cavity, but were observed in the abdominal cavity: the spleen (1 case), adrenal gland (1 case) and adipose tissue (1 case). In the group administered with cPA-19 at 0.008 mg/body, no metastatic nodules were observed in the thoracic cavity, but were observed in the abdominal cavity: the spleen (1 case). In the group administered with cPA-20 at 0.001 mg/body, metastatic nodules were observed in the thoracic cavity (1 case) and in the abdominal cavity: the diaphragm (1 case), spleen (1 case), ovary (1 case) and tissue around the glandual clitoris (1 case). In the group administered with cPA-20 at 0.004 mg/body, no metastatic nodules were observed in the thoracic cavity, but were observed in the abdominal cavity: the spleen (1 case). In the group administered with cPA-20 at 0.008 mg/body, no metastatic nodules were observed in the thoracic cavity, but were observed in the abdominal cavity: the spleen (1 case). In the cPA-5 group, no metastatic nodules were observed in the thoracic cavity, but were observed in the abdominal cavity: the spleen (3 cases). In the cPA-19 and cPA-20 groups, the number of mice having intrapleural or intraperitoneal metastatic nodules decreased as the dose increased.

### 5. Histopathological examination (Fig. 14, Table 6 and Table 7)

The results of histopathological examination are shown in Fig. 14, Table 6 and Table 7.

**Table 6: Effect of cPA-19 or cPA-20 on the number of lung metastatic lesions of B16-F0 myeloma in mice**

| Group | Dose (mg/body) | N | Number of metastatic lesions per section1) | | | |
|---|---|---|---|---|---|---|
| | | | Number of mice having metastatic lesions | Small size2) | Large size2) | Total |
| Solvent | --- | 6 | 6 | 1.3±1.5 | 1.3±1.0 | 2.7±2.4 |
| | | | | | | |
| | | | | | | |
| cPA-19 | 0.001 | 6 | 6 | 3.0±1.3 | 0.7±0.8 | 3.7±1.8 |
| | 0.004 | 6 | 2& | 0.3±0.5 | 0±0!! | 0.3±0.5! |
| | 0.008 | 6 | 0&& | 0±0! | 0±0!! | 0±0!! |
| | | | | | | |
| cPA-20 | 0.001 | 6 | 4 | 3.2±3.5 | 1.2±1.3 | 4.3±4.6 |
| | 0.004 | 6 | 1 && | 0.2±0.4 | 0±0!! | 0.2±0.4!! |
| | 0.008 | 6 | 0&& | 0±0! | 0±0!! | 0±0!! |
| | | | | | | |
| CPA-5 | 0.008 | 6 | 5 | 1.2±0.8 | 0.8±0.8 | 2.0±1.3 |

| | | | | | | |
|---|---|---|---|---|---|---|
| 1) Sections of left lobe and posterior lobe. Each value denotes meantS.D. 2) Size; Major diameter of metastatic lesion, Small size: < 0.65mm, Large size: 0.65mm≤and < 1.3mm & P<0.05, && P<0.01 vs. solvent group (Fisher's extract probability test). ! P<0.05, !! P<0.01 vs. solvent group (Wilcoxon test). | | | | | | |

**Table 7: Histopathological findings of the lungs of B16-F0 melanoma-bearing mice Findings**

| Group | Dose (mg/body) | Animal No. | Invasion of lymphocytes into melanoma metastatic lesion |
|---|---|---|---|
| Solvent | --- | 301 | - |
| | | 302 | - |
| | | 303 | - |
| | | 304 | + |
| | | 305 | - |
| | | 306 | - |
| | 0.001 | 307 | - |
| | | 308 | + |
| | | 309 | - |
| | | 310 | - |
| | | 311 | - |
| | | 312 | - |
| cPA-19 | 0.004 | 313 | * |
| | | 314 | * |
| | | 315 | - |
| | | 316 | - |
| | | 317 | * |
| | | 318 | * |
| | 0.008 | 319 | * |
| | | 320 | * |
| | | 321 | * |
| | | 322 | * |
| | | 323 | * |
| | | 324 | * |
| cPA-20 | 0.001 | 325 | - |
| | | 326 | - |
| | | 327 | * |
| | | 328 | ++ |
| | | 329 | - |
| | | 330 | * |
| | 0.004 | 331 | - |
| | | 332 | * |
| | | 333 | * |
| | | 334 | * |
| | | 335 | * |
| | | 336 | * |
| | 0.008 | 337 | * |
| | | 338 | * |
| | | 339 | * |
| | | 340 | * |
| | | 341 | * |
| | | 342 | * |
| cPA-5 | 0.008 | 343 | - |
| | | 344 | ++ |
| | | 345 | - |
| | | 346 | + |
| | | 347 | * |
| | | 348 | - |

| | | | |
|---|---|---|---|
| Grades ; -, none; +, small quantity; ++, moderate; +++,large quantity ; *: Not tested (No metastatic lesion) layer | | | |

The number of mice having metastatic lesions was 6 in the medium group. In the groups administered with cPA-19 at 0.001, 0.004 and 0.008 mg/body, the numbers were 6, 2 and 0, respectively. In the groups administered with cPA-20 at 0.001, 0.004 and 0.008 mg/body, the numbers were 4, 1 and 0, respectively. 5 mice had metastatic lesions in the cPA-5 group. Compared with the medium group, the numbers of mice having metastatic lesions significantly decreased in the groups administered with cPA-19 at 0.004 and 0.008 mg/body and cPA-20 at 0.004 and 0.008 mg/body.

Regarding the number of lung metastatic lesions, 1.3±1.5 small, 1.3±1.0 large and a total of 2.7±2.4 metastatic lesions were present in the medium group. In the group administered with cPA-19 at 0.001 mg/body, 3.0±1.3 small, 0.7±0.8 large, and a total of 3.7±1.8 metastatic lesions were present. In the group administered with cPA-19 at 0.004 mg/body, 0.3±0.5 small metastatic lesions were present, but no large metastatic lesions were observed. In the group administered with cPA-19 at 0.008 mg/body, no metastatic lesions were observed. In the group administered with cPA-20 at 0.001 mg/body, 3.2±3.5 small, 1.2±1.3 large, and a total of 4.3±4.6 metastatic lesions were present. In the group administered with cPA-20 at 0.004 mg/body, 0.2±0.4 small metastatic lesions were present, but no large metastatic lesions were observed. In the group administered with cPA-20 at 0.008 mg/body, no metastatic lesions were observed. In the cPA-5 group, 1.2±0.8 small, 0.8±0.8 large, and a total of 2.0±1.3 metastatic lesions were present. Compared with the medium group, the number of small metastatic lesions significantly decreased in the group administered with cPA-19 at 0.008 mg/body and cPA-20 at 0.008 mg/body; the number of large metastatic lesions significantly decreased in the groups administered with cPA-19 at 0.004 and 0.008 mg/body, and cPA-20 at 0.004 and 0.008 mg/body; and the total number of metastatic lesions significantly decreased in the group administered with cPA-19 at 0.004 and 0.008 mg/body and cPA-20 at 0.004 and 0.008 mg/body.

Invasion of lymphocytes into metastatic lesions was observed in 1 out of 6 cases in the medium group. In the group administered with cPA-19 at 0.001 mg/body, invasion of lymphocytes into metastatic lesions was observed in 1 out of 6 cases, but no invasions were observed in 2 cases in the group administered with the same at 0.004 mg/body. In the group administered with cPA-20 at 0.001 mg/body, invasion of lymphocytes into metastatic lesions was observed in 1 out of 4 cases, but no invasions were observed in 1 case in the group administered with the same at 0.004 mg/body. In the cPA-5 group, invasion of lymphocytes into metastatic lesions was observed in 2 out of 5 cases. There were no differences in the degree of the invasion of lymphocytes into metastatic lesions in any of the test groups.

### (Discussion)

The hematogenous metastasis that is a main pathway of cancer metastasis consists of a complex reaction cascade beginning from the detachment of cancer cells from the primary lesion and invasion into the peripheral tissue, invasion into the lymph duct and vessels, adhesion at the blood capillary floor of various organs, and finally resulting in the formation of metastatic lesions by proliferation at distant sites. By using the B16 melanoma hematogenous lung metastasis model used in this experiment, the processes following the detachment of cancer cells from the primary lesion and invasion into vessels can be examined (Poste G and Fidler I. J.: The pathogenesis of cancer metastasis. Nature 283:139-146 (1980); and Cancer Invasion/Metastasis Research Manual, ed., the Japanese Association for Metastasis Research [executive editor] Tatsuro IRIMURA, KINPODO, pp7-11, Kyoto (1994)). Lysophosphatidic acid (LPA) is one of lysophospholipid mediators (Tetsuyuki KOBAYASHI and Kimiko MUROFUSHI: the presence, metabolism and physiological functions of lysophosphatidic acid as a lysophospholipid mediator and cyclic phosphatidic acid, Protein, Nucleic Acid and Enzyme, 44, 1118-1126 (1999)). LPA is known to strongly induce an invasion in an in vitro invasion experimental system (Mutsuko MUKAI, Hitoshi AKEDO: Induction of cancer cell invasion by lysophosphatidic acid and suppression of invasion by cyclic phosphatidic acid, Protein, Nucleic Acid and Enzyme, 44: 1126-1131 (1999); Mukai M., Imamura F., Ayaki M., Shinkai K., Iwasaki T., Murakami-Murofushi K., Murofushi H., Kobayashi S., Yamamoto T., Nakamura H. and Akedo H.: Suppression of tumor invasion and metastasis by a novel lysophospharidie acid (cyclic LPA). Int., J. Cancer 81:918-922 (1999)). In contrast to LPA, cyclic phosphatidic acid (cPA), which is a structural analog of LPA, is known to strongly suppress an invasion in an in vitro invasion experimental system (Tetsuyuki KOBAYASHI and Kimiko MUROFUSHI: the presence, metabolism and physiological functions of lysophosphatidic acid as a lysophospholipid mediator and cyclic phosphatidic acid, Protein, Nucleic Acid and Enzyme, 44: 1118-1126 (1999)). Thus, the suppressing action of cPA-19 and cPA-20, which are cPA derivatives, on the lung metastasis of mouse malignant melanoma B16-F0 was examined by using the B16 melanoma hematogenous lung metastasis model.

Regarding changes over time in body weight, temporal decreases in body weight were observed in the groups administered with cPA-19 at 0.001 and 0.008 mg/body and cPA-20 at 0.001 mg/body. Such decreases in body weight were also observed in the medium group and the cPA-5 group, but not observed in the groups administered with cPA-19 at 0.004 mg/body and cPA-20 at 0.004 and 0.008 mg/body. Thus, this may not be caused by the action of the test substances. Furthermore, since a favorable body weight gain was observed afterwards, it is considered that cPA-19 and cPA-20 have no effect on changes in body weight. Furthermore, regarding the number of metastatic nodules of B16-F0 in the lungs, a sufficient number of nodules was observed in the medium group. Thus, it is considered that there was no problem in the test system employed herein. In both test groups of cPA-19 and cPA-20, administration at 0.004 mg/body caused a significant decrease in the number of metastatic nodules, and administration at 0.008 mg/body almost completely suppressed metastasis. Furthermore, similar results were obtained for the metastatic lesions inside the lung tissue or the metastasis in organs other than the lungs. This may be because cPA-19 and cPA-20 suppressed the metastasis of B16-F0. In contrast, in the cPA-5 group, a significant decrease was observed in the number of metastatic nodules, but no action of decreasing the number of metastatic lesions was observed.

From the above results, cPA-19 and cPA-20 with the dose of 0.004 and 0.008 mg/body exhibited the action of suppressing the lung metastasis of B16-F0 in this experimental model, and this action was shown to be stronger than that of cPA-5.

### Industrial Applicability

Cancer metastasis is the most significant phenomenon showing the malignant alteration of tumors, and is completed through complex processes. Above all, cancer cell invasion is a characteristic step thereof. The present invention reveals that carba-cPA has strong invasion suppressing activity and enables the provision of a novel agent for suppression of cancer metastasis. The use of the agent for suppression of cancer metastasis of the present invention greatly contributes to cancer therapy.

## Claims

1. An agent for suppression of cancer metastasis which contains a compound represented by the formula (I) as an active ingredient: 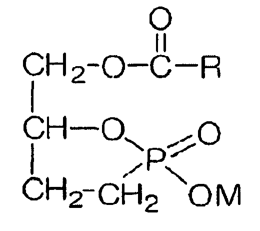 wherein R is a C₁-₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

2. The agent for suppression of cancer metastasis according to claim 1,wherein, in the formula (I), R represents -C₁₅H₃₁, -(CH₂)₇CH=CHC₆H₁₃ or -(CH₂)₇CH=CHC₈H₁₇.

## Patentansprüche

1. Mittel zur Hemmung von Krebsmetastasen, welches eine durch die Formel (I) repräsentierte Verbindung als einen Wirkstoff enthält: 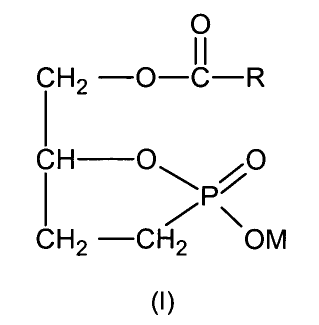 wobei R eine lineare oder verzweigte C₁₋₃₀-Alkylgruppe, eine lineare oder verzweigte C₂₋₃₀-Alkenylgruppe oder eine C₂₋₃₀-Alkinylgruppe ist, wobei diese Gruppen einen Cycloalkanring oder einen aromatischen Ring enthalten können; und M ein Wasserstoffatom oder ein Gegenkation ist.

2. Mittel zur Hemmung von Krebsmetastasen nach Anspruch 1, wobei R in der Formel (I) -C₁₅H₃₁, -(CH₂)₇CH=CHC₆H₁₃ oder -(CH₂)₇CH=CHC₈H₁₇ repräsentiert.

## Revendications

1. Agent de suppression de cancer métastasé qui contient un composé représenté par la formule (I) en tant que principe actif : 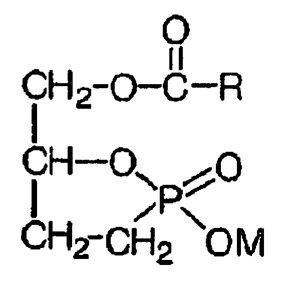 où R est un groupe alkyle linéaire ou ramifié en C₁₋₃₀, un groupe alkényle linéaire ou ramifié en C₂₋₃₀, ou un groupe alkynyle linéaire ou ramifié en C₂₋₃₀, où lesdits groupes peuvent contenir un anneau cycloalcane ou un noyau aromatique ; et M est un atome d'hydrogène ou un contrecation.

2. Agent de suppression de cancer métastasé selon la revendication 1, où dans la formule (I), R représente -C₁₅H₃₁, -(CH₂)₇CH=CHC₆H₁₃ ou -(CH₂)₇CH=CHC₈H₁₇.
